# EUROPEAN PATENT SPECIFICATION

(11) **EP 3 038 608 B1**
(45) Date of publication and mention of the grant of the patent: **03.01.2018**
(21) Application number: 14762183.3
(22) Date of filing: 28.08.2014
(51) Int. Cl.: A61K 9/70, A61K 47/12, A61K 31/565

(54) **ESTRADIOL CONTAINING TRANSDERMAL DRUG DELIVERY SYSTEMS AND COMPOSITIONS**
ESTRADIOLHALTIGE TRANSDERMALE WIRKSTOFFFREISETZUNGSSYSTEME UND ZUSAMMENSETZUNGEN
SYSTÈMES D'ADMINISTRATION TRANSDERMIQUE DE MÉDICAMENTS CONTENANT DE L'OESTRADIOL ET COMPOSITIONS

(30) Priority: 30.08.2013 US 201361872387 P
(43) Date of publication of application: 06.07.2016
(73) Proprietor: 3M Innovative Properties Company, St. Paul, MN 55133-3427 (US)
(72) Inventor: JANISCH, Andy R., Saint Paul, Minnesota 55133-3427 (US); PETERSON, Timothy A., Saint Paul, Minnesota 55133-3427 (US); DAHMEN, Keith M., Saint Paul, Minnesota 55133-3427 (US)
(74) Representative: Vossius & Partner Patentanwälte Rechtsanwälte mbB
(86) International application number: PCT/US2014/053038
(87) International publication number: WO 2015/031552

(56) References cited:
- WO-A1-95/18603
- WO-A1-2014/100599
- WO-A2-2006/036899

## Description

### FIELD

The present disclosure generally relates to transdermal drug delivery systems and compositions comprising estradiol.

### BACKGROUND

Estradiol is a natural estrogen which has limited oral effectiveness because it is rapidly metabolized by the liver to estrone and its conjugates, giving rise to higher circulating levels of estrone than estradiol. In contrast, the skin metabolizes estradiol only to a small extent. Therefore, transdermal administration produces therapeutic serum levels of estradiol with lower circulating levels of estrone and estrone conjugates, and requires smaller total doses than does oral therapy. Since estradiol has a short half-life (about one hour), transdermal administration of estradiol allows a rapid decline in blood levels after a transdermal system is removed.

Commercial estradiol containing transdermal drug delivery systems include Climara®, Menostar®, Vivelle®, Vivelle-Dot®, Estraderm®, Alora®, and Minivelle®. There yet remains a need for new, alternative estradiol transdermal drug delivery systems, including systems that increase the transdermal permeation of estradiol.

### SUMMARY

The present disclosure relates generally to transdermal drug delivery systems and compositions comprising estradiol or a salt or a solvate or hydrate thereof, and levulinic acid.
One aspect of the present disclosure provides a transdermal drug delivery system comprising a composition comprising (a) an active agent comprising an effective amount of estradiol or a salt or a solvate or hydrate thereof; and (b) levulinic acid in an amount suitable to enhance the transdermal permeation of estradiol, and (c) a pharmaceutically acceptable carrier. The carrier can comprise an adhesive matrix or a semi-solid dosage form such as a gel, cream, ointment, solution, suspension, and paste. The system can further comprise a solubilizing agent and/or one or more additional permeation enhancers.

Another aspect of the present disclosure provides a transdermal drug delivery composition comprising (a) an active agent comprising an effective amount of estradiol or a salt or a solvate or hydrate thereof; and (b) levulinic acid in an amount suitable to enhance the transdermal permeation of estradiol, and (c) a pharmaceutically acceptable carrier.

Other features and aspects of the present disclosure will become apparent by consideration of the detailed description.

### DETAILED DESCRIPTION

The present disclosure generally relates to transdermal drug delivery systems and compositions comprising estradiol. Transdermal drug delivery systems of the present invention may take any number of conventional forms. Suitable transdermal drug delivery systems include patches having gelled or liquid reservoirs, such as in U.S. Pat. No. 4,834,979 (Gale), so-called "reservoir" patches; systems containing matrix reservoirs attached to the skin by an adjacent adhesive layer, such as in U.S. Pat. No. 6,004,578 (Lee, et al.), so-called "matrix" patches; systems containing pressure-sensitive adhesive reservoirs, such as in U.S. Pat. Nos. 6,365,178 (Venkateshwaran et al.), 6,024,976 (Miranda et al.), and 6,149,935 (Chiang et al.), so-called "drug-in-adhesive" patches; and semi-solid formulations, such as gels, creams, ointments, solutions, suspensions and pastes.

Pharmaceutical carriers as used herein refers to carrier materials suitable for transdermal drug administration, and include any such materials known in the art, i.e., any liquid gel, solvent, liquid diluent, adhesive, or the like, which is nontoxic and which does not interact with other components of the composition in a deleterious manner. Carriers, which also may function as solvents in some instances, are used to provide the compositions of the invention in their preferred form. Examples include, but are not limited to, water, ethanol, propanol, isopropanol, mineral oil, silicone oil, polyethylene glycol, polypropylene glycol, liquid sugars, waxes, petroleum jelly and a variety of other oils and polymeric materials along with adhesive materials such as polyacrylate, silicone, natural and synthetic rubbers or other adhesives.

In some embodiments, the transdermal drug delivery systems are "drug-in-adhesive" type systems and as such, the estradiol and any other drugs are dispersed within an adhesive matrix that may further serve as the skin contacting adhesive. Estradiol and any other drugs may be dispersed within any portion of the adhesive matrix and may be present in dissolved and/or undissolved (i.e., particulate) form. In one embodiment the estradiol is homogeneously dispersed within the adhesive matrix. For example, estradiol may be present as small particles that are evenly (or homogeneously) mixed throughout the adhesive or the estradiol may be dissolved within the adhesive so that the concentration of dissolved estradiol is constant throughout the adhesive. In some embodiments, some or all of the estradiol is dissolved within the adhesive matrix. In some embodiments, the adhesive matrix is substantially free of undissolved estradiol, and in some embodiments, the adhesive matrix is totally free of undissolved estradiol. The presence of undissolved estradiol may be detected, for example, by examination with an optical microscope at 20 times magnification. It should be understood that where only an occasional crystal or undissolved particle is present or where less than about 1% of the total amount of estradiol is undissolved, the composition is considered to be substantially free of undissolved estradiol.

Examples of suitable types of polymers for use in the transdermal drug delivery systems of the present invention include acrylates, natural and synthetic rubbers such as polyisobutylenes, polysiloxanes, polyurethanes, and other polymers known in the art to be useful as components of pressure-sensitive skin adhesive compositions. The polymers can be present alone or in combination.

In some embodiments the adhesive matrix is a pressure-sensitive adhesive including, for example, acrylates, polyisobutylenes, silicone polymers, and mixtures thereof. Examples of suitable polyisobutylene pressure-sensitive adhesives are described in U.S. Pat. No. 5,985,317 (Venkateshwaran et al.). In some embodiments, the adhesive matrix is a blend of an acrylate polymer and a silicone polymer. In some embodiments, the adhesive matrix is a blend of an acrylate copolymer and a silicone polymer. Examples of suitable acrylate and silicone polymer pressure-sensitive adhesives, and mixtures thereof, are described in U.S. Pat. No. 5,474,783 (Miranda). The adhesive may optionally contain other additives, such as tackifiers, plasticizers, anti-oxidants, colorants, crystallization inhibitors, and the like.In some embodiments, the pressure-sensitive adhesive comprises acrylate polymers and/or copolymers. In some embodiments, the adhesive comprises the copolymerization product of at least one first monomer selected from the group consisting C₄ to C₁₂ alkyl acrylate monomers, C₄ to C₁₂ alkyl methacrylate monomers, and combinations thereof; and at least one second monomer selected from the group consisting of acrylamide, N,N-diethylacrylamide, methacrylamide, vinyl acetate, vinyl alcohol, N-vinyl-2-pyrrolidone, 2-hydroxyethyl acrylate, 2-hydroxyethyl methacrylate, hydroxpropyl acrylate, glyceryl acrylate, 2-ethoxyethyl acrylate, 2-ethoxyethoxyethyl acrylate, tetrahydrofurfuryl acrylate, acrylic acid, methacrylic acid, pyrrolidonylethyl acrylate, 2-carboxyethyl acrylate, and combinations thereof. In some embodiments, the at least one first monomer is selected from the group consisting of isooctyl acrylate, 2-ethylhexyl acrylate, cyclohexyl acrylate, 2-methylbutyl acrylate, butyl acrylate, and combinations thereof. In some embodiments, the at least one second monomer is selected from the group consisting of acrylamide, vinyl acetate, and a combinations thereof. In some embodiments, the amount of the at least one first monomer is typically between about 40% and about 98% by weight of the resulting copolymer. In some embodiments, the amount of the at least one first monomer is between about 60% and about 95% by weight of the resulting copolymer composition, and in some embodiments, the amount of the at least one first monomer is between about 70% and about 90% by weight of the resulting copolymer composition. In some embodiments, the amount of the at least one second monomer is between about 2% and about 60% weight of the copolymer composition. In some embodiments, the amount of the at least one second monomer is between about 3% and about 40% by weight of the copolymer composition, and in some embodiments, the amount of the at least one second monomer is between about 4% and about 30% by weight of the copolymer composition. In some embodiments, the copolymers comprising the pressure sensitive adhesive may optionally further comprise other radically-polymerizable monomers that are well known in the art. In some embodiments, the copolymers comprising the pressure sensitive adhesive may optionally further comprise a substantially linear macromonomer copolymerizable with the other monomers. Suitable macromonomers include polymethylmethacrylate, styrene/acrylonitrile copolymer, polyether, and polystyrene macromonomers.

In some embodiments, including any one of the above embodiments, the adhesive comprises the co-polymerization product of isooctyl acrylate, acrylamide, and vinyl acetate. In some embodiments, the isooctylacrylate is 70-80%, the acrylamide is 3-7%, and the vinyl acetate is 15-25% of the copolymerization product on a weight basis. In some embodiments, the isooctyl acrylate/acrylamide/vinyl acetate weight ratio is 75/5/20. Acrylate adhesives as described in U.S. Patent Application Publication 2013-0122079 (Dizio et al.) are suitable for use in the present invention, including adhesives that have undergone further processing to remove unreacted residual monomers.

Additional examples of adhesives which may be used in the present systems and compositions include copolymers of isooctyl acrylate/acrylamide (for example, about 93/7 weight ratio); copolymers of ethylhexyl acrylate, butyl acrylate, and vinyl acetate; DUROTAK 87-2353, 387-2353, 387-2051, and 387-2052 (National Starch and Chemical Company, Zutphen, Holland); PLASTOID B (Rohm, Darmstadt, Germany); and EUDRAGIT E 100 (Rohm, Darmstadt, Germany).

Transdermal drug delivery systems and compositions of the present invention comprise estradiol or a salt or a solvate or hydrate thereof. For ease of description, it should be understood that general reference to "estradiol" throughout the specification is understood to encompass estradiol or a salt or a solvate or hydrate thereof unless a specific indication of the form is given. Estradiol is described chemically as estra-1,3,5(10)-triene-3,17β-diol and sometimes referred to as 17β-estradiol or oestradiol. The estradiol is present in the composition or system in a pharmaceutically effective amount. In some embodiments, the estradiol is present in an amount of from about 0.2 to 12 percent by weight of the total weight of the composition. In some embodiments, the estradiol is present in an amount of from about 1 to 5 percent by weight. In some embodiments, In some embodiments, the estradiol is present in an amount of from about 2 to 3.5 percent by weight. In some embodiments, estradiol is present as estradiol hemihydrate.

In some embodiments, transdermal drug delivery systems and compositions of the present invention comprise levulinic acid. In particular, it has been found that levulinic acid provides for a surprising increase in the transdermal permeation of estradiol. In some embodiments, the amount of levulinic acid is more than about 4%, more than about 7%, or more than about 10% by weight of the composition. In some embodiments, the amount of levulinic acid is less than about 25%, less than about 20%, or less than about 15% by weight of the composition. In some embodiments, the amount of levulinic acid is between about 7% and about 20%, by weight of the total weight of the composition. In some embodiments, the amount of levulinic acid is between about 10% and about 15%, by weight of the total weight of the composition.

In some embodiments, transdermal drug delivery systems and compositions of the present invention comprise a solubilizing agent. A solubilizing agent is a generally low molecular weight compound (i.e., typically with a molecular weight of 2000 g/mol or less) that may be added to an adhesive in order to increase the solubility of a drug within the system or composition. The solubility of drug, such as estradiol, will be greater in the solubilizing agent than it is in the adhesive, and thus a mixture of adhesive and solubilizing agent will be able to dissolve a higher concentration of drug than the adhesive can alone. In some embodiments, the solubility of estradiol in the solubilizing agent is greater than 5% by weight. In some embodiments, the solubility of estradiol in the solubilizing agent is greater than 10% by weight, and in some embodiments, greater than 20% by weight. Solubility of estradiol may be determined by any of a number of conventional methods. For example, solubility in a liquid solubilizing agent may be determined by adding an excess of drug to the liquid and mixing to allow the maximum amount of drug to dissolve into the liquid. The excess, undissolved drug is then filtered from the solution and the solution is analyzed for dissolved drug concentration. Solubility in an adhesive matrix may be determined, for example, by preparing sheets of adhesive with varying concentrations of drug and observing them over time to see if drug crystallizes. The solubility is then considered to be the highest concentration of drug that can be added to the sheet without leading to crystallization on extended storage (e.g., 3-, 6-, 12-, or 24-month storage).

In some embodiments, the amount of solubilizing agent is between about 1 and about 20%, by weight of the total weight of the composition. In some embodiments, the amount of solubilizing agent is between about 2 and about 6%, by weight of the total weight of the composition.

Suitable solubilizing agents include butylene glycol, diethylene glycol methyl ether, dipropylene glycol, propylene glycol, and mixtures thereof. In some embodiments the solubilizer is dipropylene glycol. In some embodiments, the amount of dipropylene glycol is more than about 1%, more than about 2%, or more than about 3% by weight of the composition. In some embodiments, the amount of dipropylene glycol is less than about 10%, less than about 6%, or less than about 4% by weight of the composition.

In some embodiments, the solubilizing agent is polyvinylpyrrolidone (PVP). In some embodiments, the PVP is PVP K-30. In some embodiments, the amount of PVP or PVP K-30 is between about 1 and about 20%, by weight of the total weight of the composition. In some embodiments, the amount of PVP or PVP K-30 is between about 2 and about 12%, by weight of the total weight of the composition. In some embodiments, the amount of PVP is more than about 1%, more than about 2%, or more than about 4% by weight of the composition. In some embodiments, the amount of PVP is less than about 20%, less than about 15%, or less than about 10% by weight of the composition.

In some embodiments, transdermal drug delivery systems and compositions of the present invention comprise one or more permeation enhancers in addition to levulinic acid. Exemplary materials include C₈-C₃₆ fatty acids such as isostearic acid, octanoic acid, and oleic acid; C₈-C₃₆ fatty alcohols such as oleyl alcohol and lauryl alcohol; lower alkyl esters of C₈-C₃₆ fatty acids such as ethyl oleate, isopropyl myristate, butyl stearate, and methyl laurate; di(lower) alkyl esters of C₆-C₈ diacids such as diisopropyl adipate; monoglycerides of C₈-C₃₆ fatty acids such as glyceryl monolaurate; tetraglycol (tetrahydrofurfuryl alcohol polyethylene glycol ether); tetraethylene glycol (ethanol,2,2'-(oxybis(ethylenoxy))diglycol); C₆-C₃₆ alkyl pyrrolidone carboxylates; polyethylene glycol; propylene glycol; 2-(2-ethoxyethoxy)ethanol; diethylene glycol monomethyl ether; N,N-dimethyldodecylamine N-oxide; and combinations of the foregoing. Alkylaryl ethers of polyethylene oxide, polyethylene oxide monomethyl ethers, and polyethylene oxide dimethyl ethers are also suitable. Terpenes are another useful class of enhancers, including pinene, *d*-limonene, carene, terpineol, terpinen-4-ol, carveol, carvone, pulegone, piperitone, menthone, menthol, neomenthol, thymol, camphor, borneol, citral, ionone, and cineole, alone or in any combination. In some embodiments, additional permeation enhancers include lauryl lactate, oleic acid, and mixtures thereof. In some embodiments, additional permeation enhancers may include sorbitan monooleate.

In some embodiments, the amount of additional permeation enhancer (i.e., permeation enhancer not including levulinic acid) is between about 1 and about 30% by weight of the total weight of the composition. In some embodiments, the amount of additional permeation enhancer (i.e., permeation enhancer not including levulinic acid) is between about 4 and about 20%, by weight of the total weight of the composition. In some embodiments the amount of additional permeation enhancer (i.e., permeation enhancer not including levulinic acid) is more than about 2%, more than about 4%, or more than about 6% by weight of the total weight of the composition. In some embodiments the amount of additional permeation enhancer (i.e., permeation enhancer not including levulinic acid) is less than about 12%, less than about 10%, or less than about 8% by weight of the total weight of the composition.

The physical properties desirable in a transdermal drug delivery system are well known to those skilled in the art. For example, in adhesive patch systems it is desirable to have sufficiently little cold flow such that a system of the invention is stable to flow upon storage. It is also preferred that it adhere well to the skin and release cleanly from the skin. In order to achieve resistance to cold flow, preferred levels of skin adhesion and clean release, the amount and structure of the comonomers in the copolymer, the inherent viscosity of the copolymer, and the amount and type of adjuvants, such as permeation enhancers and solubilizers, are selected by those skilled in the art such that the adhesive layer(s) obtain the desired balance of these properties.

In some embodiments, the total amount of permeation enhancer (including levulinic acid and any optional permeation enhancers) and solubilizing agent is more than about 15% by weight of the total weight of the composition.. In some embodiments, the total amount of permeation enhancer (including levulinic acid and any optional permeation enhancers) and solubilizing agent is equal to or more than about 20% by weight of the total weight of the composition. In some embodiments, the total amount of permeation enhancer (including levulinic acid and any optional permeation enhancers) and solubilizing agent is less than about 30% by weight of the total weight of the composition. In some embodiments, the total amount of permeation enhancer (including levulinic acid and any optional permeation enhancers) and solubilizing agent is less than about 25% by weight of the total weight of the composition.

In some embodiments, compositions of the present invention comprise a combination of levulinic acid and a solubilizer in amounts as described above. In some embodiments, compositions of the present invention comprise a combination of levulinic acid and an additional permeation enhancer in amounts as described above. In some embodiments, compositions of the present invention comprise a combination of levulinic acid, a solubilizer, and an additional permeation enhancer in amounts as described above. In some embodiments, compositions of the present invention comprise a combination of levulinic acid, a solubilizer, and two additional permeation enhancers in amounts as described above. In some embodiments, compositions of the present invention comprise a combination of levulinic acid, dipropylene glycol, and lauryl lactate in amounts as described above. In some embodiments, compositions of the present invention comprise a combination of levulinic acid, dipropylene glycol, and oleic acid in amounts as described above. In some embodiments, compositions of the present invention comprises a combination of levulinic acid, dipropylene glycol, oleic acid, and lauryl lactate in amounts as described above.

In some embodiments, transdermal drug delivery systems of the invention also comprise a backing. The backing is flexible such that the system conforms to the skin. Suitable backing materials include conventional flexible backing materials used for pressure-sensitive adhesive tapes, such as polyethylene, particularly low density polyethylene, linear low density polyethylene, metallocene polyethylenes, high density polyethylene, polypropylene, polyolefin blends, polyesters such as polyethylene terephthalate, randomly oriented nylon fibers, ethylene-vinyl acetate copolymer, polyurethane, natural fibers such as rayon and mixtures thereof. Backings that are layered, such as polyethylene terephthalate-aluminum-polyethylene composites, are also suitable. The backing should be substantially inert to the components of the adhesive layer.

In some embodiments, transdermal drug delivery systems of the invention are prepared by combining adhesive, estradiol, levulinic acid and any optional solubilizers or permeation enhancers with an organic solvent (e.g., ethyl acetate, isopropanol, methanol, acetone, 2-butanone, ethanol, toluene, alkanes, and mixtures thereof) to provide a coating composition. The mixture is shaken or stirred until a homogeneous coating composition is obtained. The resulting composition is then applied to a release liner using conventional coating methods (e.g., knife coating or extrusion die coating) to provide a predetermined uniform thickness of coating composition. Suitable release liners include conventional release liners comprising a known sheet material such as a polyester web, a polyethylene web, a polystyrene web, or a polyethylene-coated paper coated with a suitable fluoropolymer or silicone based coating. The release liner that has been coated with the composition is then dried and laminated onto a backing using conventional methods. In some embodiments this initial release liner, sometimes referred to as the "coating" release liner, may be removed and replaced with another release liner.

In some embodiments, the transdermal drug delivery systems of the invention can be made or packaged in the form of an article such as a tape, a patch, a sheet, a dressing, a sachet, a tube, or any other form known to those skilled in the art. In some embodiments, the system will be in the form of a patch of a size suitable to deliver a preselected amount of estradiol through the skin. In some embodiments, the system will have a surface area of more than about 1 cm², more than about 2 cm², or more than about 4 cm². In some embodiments, the system will have a surface area of less than about 40 cm², less than about 20 cm², or less than about 10 cm². In some embodiments, the system will have a surface area of about 1 cm² to about 40 cm², about 2 cm² to about 20 cm², or about 2 cm² to about 10 cm². In some embodiments, the thickness of the adhesive layer will be more than about 25 microns, or more than about 50 microns. In some embodiments, the thickness of the adhesive layer will be less than about 150 microns, or less than about 120 microns.

In some embodiments, the transdermal drug delivery system comprises more than one discrete adhesive layer. For example, the transdermal drug delivery system may comprise two adhesive layers, including a first adhesive layer that serves as a skin-contacting adhesive layer and a second adhesive layer sandwiched between the first adhesive layer and the backing. In some embodiments, the second adhesive layer is thicker than the first adhesive layer and serves as a drug reservoir and is referred to as the "reservoir" layer.

In some embodiments, the first adhesive layer may be a rate-controlling layer. That is, the rate-controlling layer serves to control the rate of delivery of the drug to the subject and to adhere the system to the subject's skin. Thus the presence of the rate-controlling layer in the system may change the skin permeation profile of the system compared to a like system where the rate-controlling layer is identical in composition to the reservoir layer. This control of rate of delivery of the drug may be due to differences in the affinity of the drug for the two different layers and differences in the rate of diffusion of the drug through the two different layers. These differences in affinity and/or diffusion of the drug in the two layers, as well as the relative thickness of the layers, allows the rate of delivery of the drug to be controlled. This system is referred to as an "adhesive rate controlled system".

In some embodiments of the adhesive rate controlled system, the two layers are selected so that the second (rate-controlling) layer has a lower affinity for the drug than the first (reservoir) layer. By "lower affinity" is meant that the drug preferentially resides in the reservoir layer, so that when the system is at equilibrium the weight percentage of drug in the reservoir layer is greater than the weight percentage of drug in the rate controlling layer. The difference in the affinity of the two layers for the drug, as well as the relative thickness of the layers, allows the rate of delivery of the drug to be controlled.

The rate-controlling layer differs in composition from the reservoir layer. In some embodiments, the first and second layers may contain, for example, different types and amounts of polymers, including polymers that differ in their extent of reaction, crosslinking, branching, and copolymer sequences. In some embodiments, pressure sensitive adhesives of the rate-controlling layer comprise polymers selected from the group consisting of acrylates, natural rubbers, synthetic rubbers such as polyisobutylenes, polyisoprenes, styrene block copolymers and silicone polymers. In some embodiments, the pressure sensitive adhesives of the rate-controlling layer comprise silicone polymers.

In some embodiments, the drug has a higher solubility in the second adhesive layer than in the first adhesive layer, such that there is a higher concentration of drug in the second adhesive layer than in the first adhesive layer. In some embodiments, the thickness of the first adhesive layer will be more than about 10 microns, or more than about 20 microns. In some embodiments, the thickness of the first adhesive layer will be less than about 50 microns, or less than about 40 microns. In some embodiments, the thickness of the second adhesive layer will be more than about 25 microns, or more than about 50 microns. In some embodiments, the thickness of the second adhesive layer will be less than about 150 microns, or less than about 120 microns.

In some embodiments, the transdermal drug delivery system comprises a rate-controlling membrane. In some embodiments, this rate-controlling membrane is adhered on one side to a first adhesive layer and on the opposing side to a second adhesive layer. The first and second adhesives layers may be as described above. The membrane is selected such that it is rate-controlling, i.e., the presence of the membrane in the system may change the skin permeation profile of the system compared to a like system not having the membrane. Suitable membranes include continuous film membranes and microporous membranes. In some embodiments, the membranes are continuous film membranes prepared from ethylene:vinyl acetate copolymers containing from about 0.5 to about 28 wt-% vinyl acetate. In some embodiments, membranes are continuous film membranes prepared from ethylene:vinyl acetate copolymers containing about 2 to about 9 wt-% vinyl acetate. In some embodiments, the membrane thickness will generally be from about 25 microns to about 100 microns. In some embodiments, the membrane thickness will be about 50 microns.

Because the delivery rate of the drug is controlled by the membrane, a variety of pressure sensitive adhesives that have a range of affinities for the drug may be used in the skin contacting layer. The pressure sensitive adhesive used in the skin contacting layer can be the same as or different from the transdermal drug delivery composition used in the reservoir layer. Pressure sensitive adhesives used in the skin contacting layer may comprise polymers selected from the group consisting of acrylates, natural rubbers, synthetic rubbers such as polyisobutylenes, polyisoprenes, styrene block copolymers and silicone polymers. In some embodiments, the pressure sensitive adhesive used in the skin contacting layer is the same as the transdermal drug delivery composition used in the reservoir layer.

In some embodiments, the skin contacting layer can initially contain drug in a concentration similar to that of the reservoir layer or the skin contacting layer can contain no drug, since it is expected that over time drug will diffuse from the reservoir layer into the skin contacting layer.

### EMBODIMENTS

Embodiment 1 is a transdermal drug delivery system comprising:
a composition comprising:
   (a) an active agent comprising an effective amount of estradiol or a salt or a solvate or hydrate thereof; and
   (b) levulinic acid in an amount suitable to enhance the transdermal permeation of estradiol, and
   (c) a pharmaceutically acceptable carrier.

Embodiment 2 is a transdermal drug delivery system according to Embodiment 1 wherein the carrier is an adhesive matrix.

Embodiment 3 is a transdermal drug delivery system according to Embodiment 2 wherein the adhesive matrix comprises an acrylate copolymer.

Embodiment 4 is a transdermal drug delivery system according to Embodiments 2 or 3 and further comprising a backing film.

Embodiment 5 is a transdermal drug delivery system according to Embodiment 4 having a surface area of about 2 cm² to about 10 cm².

Embodiment 6 is a transdermal drug delivery system according to any one of Embodiments 2 to 5 wherein the adhesive matrix is a reservoir layer and further comprising a skin-contacting adhesive layer that is distinct from the reservoir layer.

Embodiment 7 is a transdermal drug delivery system according to Embodiment 6 wherein the skin-contacting adhesive layer comprises a silicone adhesive.

Embodiment 8 is a transdermal drug delivery system according to Embodiment 1, wherein the carrier is a semi-solid dosage form.

Embodiment 9 is a transdermal drug delivery system according to Embodiment 8 wherein the carrier is selected from the group consisting of gel, cream, ointment, solution, suspension, and paste. Embodiment 10 is a transdermal drug delivery system according to any of the preceding Embodiments and further comprising a solubilizing agent.

Embodiment 11 is a transdermal drug delivery system according to Embodiment 10 wherein the solubilizing agent is selected from the group consisting of butylene glycol, diethylene glycol methyl ether, dipropylene glycol, propylene glycol, and mixtures thereof.

Embodiment 12 is a transdermal drug delivery system according to Embodiment 10 wherein the solubilizing agent is dipropylene glycol.

Embodiment 13 is a transdermal drug delivery system according to any one of Embodiments 10 to 12 wherein the amount of solubilizing agent is between about 2 and about 6 percent by weight of the composition.

Embodiment 14 is a transdermal drug delivery system according to any of the preceding Embodiments wherein the estradiol is homogeneously dispersed within the composition.

Embodiment 15 is a transdermal drug delivery system according to any of the preceding Embodiments and further comprising one or more additional permeation enhancers selected from the group consisting of lauryl lactate, oleic acid, and mixtures thereof.

Embodiment 16 is a transdermal drug delivery system according to Embodiment 15 wherein the amount of additional permeation enhancer is between about 4 and about 8 percent by weight of the composition.

Embodiment 17 is a transdermal drug delivery system according to any of the preceding Embodiments wherein the amount of estradiol is between about 2 and about 5 percent by weight of the composition.

Embodiment 18 is a transdermal drug delivery system according to any of the preceding Embodiments wherein the active agent is estradiol hemihydrate.

Embodiment 19 is a transdermal drug delivery system according to any of the preceding Embodiments wherein the amount of levulinic acid is between 5 and 25 percent by weight of the composition.

Embodiment 20 is a transdermal drug delivery system according to any of Embodiments 2-7, wherein the adhesive comprises the co-polymerization product of isooctyl acrylate, acrylamide, and vinyl acetate.

Embodiment 21 is a transdermal drug delivery composition comprising:
(a) an active agent comprising an effective amount of estradiol or a salt or a solvate or hydrate thereof; and
(b) levulinic acid in an amount suitable to enhance the transdermal permeation of estradiol, and
(c) a pharmaceutically acceptable carrier.

Embodiment 22 is a a transdermal adhesive composition comprising estradiol and levulinic acid.

Embodiment 23 is a transdermal drug delivery system according to Embodiment 2 wherein the adhesive matrix comprises an acrylate copolymer or a blend of an acrylate copolymer and a silicone polymer.

### MATERIALS

### Adhesive:

The adhesive composition used in making the transdermal drug delivery (TDD) systems of Examples 1-4 was a solvent washed isooctyl acrylate/acrylamide/vinyl acetate (75/5/20) copolymer that was prepared according to the procedure described in U.S. Patent Publication No. 2013/0122079 (Dizio et al.) for the preparation of Copolymer B.

The adhesive composition used in making the transdermal drug delivery (TDD) systems of Examples 5-6 was a 26/74 weight ratio blend (based on dry polymer) of the isooctyl acrylate/acrylamide/vinyl acetate (75/5/20) adhesive (described above for Examples 1-4) and AC7-4302 silicone adhesive (available from the Dow Coming Corporation, Midland, MI). AC7-4302 silicone adhesive was solvated at approximately 60 weight % solids in ethyl acetate.

### EXAMPLES

### Example 1. TDD Systems

Twenty-nine different transdermal drug delivery (TDD) systems were prepared each containing estradiol hemihydrate (CAS Number 35380-71-3, molecular formula = C₁₈H₂₄O_{2˙}0.5 H₂O) in a different drug in adhesive (DIA) formulation. For each TDD system the individual formulation components were combined with the isooctyl acrylate/acrylamide/vinyl acetate (75/5/20) adhesive copolymer (35% solids in a solvent system of about 70/30 ethyl acetate/ methanol). The formulation mixture was gently agitated with a laboratory roller mixer to achieve a uniform distribution of the formulation components. The formulation was then knife coated onto a ScotchPak™ 9744 polyester film release liner (available from 3M Company, Maplewood, MN) at a setting to deliver a wet coating thickness of about 18 mil (0.46 mm). The coated liner was oven dried (10 minutes at 60 °C) and then laminated onto a CoTran™ 9722 polyolefin monolayer film backing (available from 3M Company). The dry coating weight for each of the TDD Systems 1-29 was about10 mg/cm². The finished TDD systems were die cut as individual circles with adhesive containing surface areas of 1 cm². The nominal concentrations of the formulation components in each finished TDD system are listed in Table 1.

**Table 1.**

| TDD System Number | Concentration in the Adhesive Formulation of the Finished TDD System (Percent by Weight) | | | | |
|---|---|---|---|---|---|
| | Estradiol Hemihydrate | Levulinic Acid | Lauryl Lactate | Oleic Acid | Dipropylene Glycol |
| 1 | 3.0% | 5.0% | 15.0% | 0% | 0% |
| 2 | 3.0% | 8.7% | 3.7% | 3.7% | 3.7% |
| 3 | 3.0% | 5.0% | 0% | 15.0% | 0% |
| 4 | 3.0% | 14.4% | 1.9% | 1.9% | 1.9% |
| 5 | 3.0% | 8.7% | 3.7% | 0% | 7.5% |
| 6 | 3.0% | 7.5% | 2.5% | 2.5% | 7.5% |
| 7 | 3.0% | 8.7% | 3.7% | 3.7% | 3.7% |
| 8 | 3.0% | 6.9% | 9.4% | 1.9% | 1.9% |
| 9 | 3.0% | 6.9% | 5.6% | 1.9% | 5.6% |
| 10 | 3.0% | 16.2% | 0% | 0% | 3.8% |
| 11 | 3.0% | 12.5% | 7.5% | 0% | 0% |
| 12 | 3.0% | 6.9% | 1.9% | 9.4% | 1.9% |
| 13 | 3.0% | 5.0% | 7.5% | 0% | 7.5% |
| 14 | 3.0% | 5.0% | 11.2% | 0% | 3.8% |
| 15 | 3.0% | 6.9% | 1.9% | 5.6% | 5.6% |
| 16 | 3.0% | 5.0% | 3.8% | 3.8% | 7.5% |
| 17 | 3.0% | 10.6% | 0% | 5.6% | 3.8% |
| 18 | 3.0% | 10.6% | 5.6% | 0% | 3.8% |
| 19 | 3.0% | 5.0% | 7.5% | 7.5% | 0% |
| 20 | 3.0% | 5.0% | 0% | 7.5% | 7.5% |
| 21 | 3.0% | 12.5% | 0% | 0% | 7.5% |
| 22 | 3.0% | 12.5% | 0% | 7.5% | 0% |
| 23 | 3.0% | 20.0% | 0% | 0% | 0% |
| 24 | 3.0% | 5.0% | 0% | 11.2% | 3.8% |
| 25 | 3.0% | 8.8% | 3.8% | 3.8% | 3.8% |
| 26 | 3.0% | 10.6% | 1.9% | 1.9% | 5.6% |
| 27 | 3.0% | 10.0% | 5.0% | 5.0% | 0% |
| 28 | 3.0% | 8.7% | 0% | 3.8% | 7.5% |
| 29 | 3.0% | 5.0% | 5.6% | 5.6% | 3.8% |

### Example 2. Skin Permeation Study

*In vitro* skin permeation of estadiol hemihydrate from each TDD system of Example 1 was determined using a vertical diffusion cell. Dermatomed human cadaver skin was used as the diffusion membrane. The diffusion surface between the compartments was 1 cm² and matched the circular surface of the TDD system. The TDD system was applied to the epidermal side of the human cadaver skin sample and pressed to cause uniform contact with the skin sample. The skin sample was then positioned and aligned across the orifice of the receptor compartment of the diffusion cell so that the side of the skin sample opposite from the attached TDD system faced the receptor compartment. The diffusion cell was assembled by clamping the upper donor and lower receptor compartments together. The receptor fluid was prepared as polyethylene glycol 400 (PEG400) (20% volume/volume) in phosphate buffered saline (PBS) (pH7.4). The total volume of the receptor compartment (about 5 mL) was filled with receptor fluid so that the skin sample was in contact with the fluid. The receptor fluid was stirred using a magnetic stir bar. The diffusion cell was housed in an enclosed chamber that was maintained at 32 °C. At time points of 6, 12, 24, 48, and 72 hours the entire receptor fluid solution was decanted from the receptor compartment through the sampling port. The receptor compartment was then refilled with fresh receptor fluid. The sampling port was covered except during the periods when receptor fluid was being transferred. The receptor fluid that was removed was filtered (0.45 micron filter) and analyzed for estradiol hemihydrate content using reversed phase high performance liquid chromatography (Agilent 1200 HPLC instrument equipped with a ultraviolet detector set at 225 nm, Agilent Technologies, Santa Clara, CA). The analytical column used was a Zorbax XDB-C18 column with a 50 mm length, 4.6 mm inner diameter, and 5 micron particle size (Agilent Technologies). The column was maintained at 40 °C. An isocratic elution was conducted with the mobile phase consisting of acetonitrile, methanol, and water in a ratio of 28:7:65. The flow rate was 2 mL/minute. The cumulative amount of estradiol hemihydrate delivered from the TDD system to the receptor compartment (µg) was determined at each time point. For each system a total of 4 replicates were analyzed and the mean value is reported in Table 2.

**Table 2.**

| TDD System Number | Cumulative Amount of Estradiol Hemihydrate (µg) Delivered to the Receptor Compartment at Sampling Timepoints (mean value reported with n=4) | | | | |
|---|---|---|---|---|---|
| | 6 hours | 12 hours | 24 hours | 48 hours | 72 hours |
| 1 | 2.8 | 6.3 | 11.0 | 16.4 | 19.9 |
| 2 | 3.5 | 7.3 | 13.4 | 20.6 | 25.2 |
| 3 | 1.9 | 4.7 | 9.5 | 16.2 | 20.9 |
| 4 | 2.0 | 5.4 | 11.1 | 19.0 | 24.0 |
| 5 | 2.2 | 5.2 | 10.0 | 16.8 | 21.5 |
| 6 | 2.1 | 5.2 | 10.6 | 18.6 | 24.8 |
| 7 | 2.7 | 6.0 | 11.3 | 18.4 | 23.3 |
| 8 | 2.8 | 6.4 | 12.1 | 18.8 | 23.2 |
| 9 | 1.9 | 4.7 | 9.7 | 16.8 | 21.9 |
| 10 | 1.9 | 4.5 | 9.2 | 16.3 | 21.8 |
| 11 | 3.0 | 6.3 | 12.3 | 19.4 | 23.9 |
| 12 | 2.2 | 5.3 | 10.6 | 17.2 | 21.6 |
| 13 | 2.0 | 5.1 | 10.3 | 17.6 | 22.8 |
| 14 | 2.5 | 5.4 | 10.2 | 16.4 | 20.8 |
| 15 | 1.5 | 4.0 | 8.6 | 14.9 | 19.5 |
| 16 | 1.4 | 4.0 | 8.6 | 15.5 | 21.1 |
| 17 | 2.5 | 6.0 | 11.8 | 19.9 | 26.0 |
| 18 | 2.5 | 5.6 | 10.4 | 16.7 | 21.3 |
| 19 | 2.0 | 5.3 | 9.9 | 15.6 | 19.2 |
| 20 | 1.3 | 3.5 | 7.5 | 12.9 | 16.7 |
| 21 | 2.6 | 5.9 | 11.1 | 19.1 | 25.5 |
| 22 | 3.1 | 7.5 | 14.2 | 22.5 | 28.3 |
| 23 | 2.4 | 6.1 | 12.2 | 19.9 | 25.0 |
| 24 | 2.3 | 5.1 | 9.5 | 15.7 | 20.3 |
| 25 | 2.5 | 6.0 | 11.4 | 18.8 | 24.5 |
| 26 | 2.4 | 5.6 | 10.6 | 17.3 | 22.2 |
| 27 | 3.0 | 7.1 | 13.2 | 21.1 | 26.9 |
| 28 | 1.8 | 4.8 | 10.2 | 18.0 | 23.6 |
| 29 | 2.6 | 6.2 | 12.1 | 19.8 | 25.6 |

### Example 3. TDD Systems

Ten different transdermal drug delivery systems (TDD Systems 30-39) were prepared each with a different drug in adhesive (DIA) formulation. For each TDD system the individual formulation components were combined with the isooctyl acrylate/acrylamide/vinyl acetate (75/5/20) adhesive copolymer (35% solids in a solvent system of about 70/30 ethyl acetate/methanol). The formulation mixture was gently agitated with a laboratory roller mixer to achieve a uniform distribution of the formulation components. The formulation was then knife coated onto a ScotchPak™ 9744 polyester film release liner (available from 3M Company, Maplewood, MN) at a setting to deliver a wet coating thickness of about 18 mil (0.46 mm). The coated liner was oven dried (10 minutes at 60 °C) and then laminated onto a CoTran™ 9722 polyolefin monolayer film backing (available from 3M Company). The dry coating weight for each of the TDD Systems 30-39 was about 10 mg/cm². The finished TDD systems were die cut as individual circles with adhesive containing surface areas of 1 cm². The nominal concentrations of the formulation components in each finished TDD system are listed in Table 3. For Comparative Example 1, a TDD system was prepared as described above in which no levulinic acid was incorporated in the DIA formulation.

**Table 3.**

| TDD System Number | Concentration in the Adhesive Formulation of the Finished TDD System (Percent by Weight) | | | | | | | |
|---|---|---|---|---|---|---|---|---|
| | Estradiol Hemihydrate | Levulinic Acid | Lauryl Lactate | Oleic Acid | Methyl Laurate | Oleyl Alcohol | Sorbitan Monooleate | Dipropylene Glycol |
| 30 | 3.0% | 10.0% | 10.0% | 0% | 0% | 0% | 0% | 0% |
| 31 | 3.0% | 10.0% | 0% | 0% | 10.0% | 0% | 0% | 0% |
| 32 | 3.0% | 10.0% | 0% | 0% | 0% | 10.0% | 0% | 0% |
| 33 | 3.0% | 10.0% | 0% | 0% | 0% | 0% | 10.0% | 0% |
| 34 | 3.0% | 10.0% | 0% | 10.0% | 0% | 0% | 0% | 0% |
| 35 | 4.0% | 10.0% | 0% | 0% | 0% | 0% | 0% | 10.0% |
| 36 | 4.0% | 15.0% | 0% | 0% | 0% | 0% | 0% | 5.0% |
| 37 | 3.0% | 5.0% | 0% | 0% | 0% | 0% | 0% | 0% |
| 38 | 3.0% | 10.0% | 0% | 0% | 0% | 0% | 0% | 0% |
| 39 | 3.0% | 20.0% | 0% | 0% | 0% | 0% | 0% | 0% |
| Comparative Example 1 | 3.0% | 0% | 0% | 0% | 0% | 0% | 0% | 0% |

### Example 4. Skin Permeation Study

*In vitro* skin permeation of estadiol hemihydrate from each TDD system of Example 3 was determined using the same procedure as described in Example 2. The only change was that the receptor fluid was prepared as PEG400 (40% volume/volume) in PBS (pH 7.4), instead of PEG400 (40% volume/volume) in PBS (pH 7.4). The cumulative amount of estradiol hemihydrate delivered from the TDD system to the receptor compartment (µg) was determined at each time point. For each system a total of 4 replicates were analyzed and the mean value is reported in Table 4.

**Table 4.**

| TDD System Number | Cumulative Amount of Estradiol Hemihydrate (µg) Delivered to the Receptor Compartment at Sampling Timepoints (mean value reported with n=4) | | | | |
|---|---|---|---|---|---|
| | 6 hours | 12 hours | 24 hours | 48 hours | 72 hours |
| 30 | 2.1 | 6.5 | 11.9 | 17.9 | 22.5 |
| 31 | 1.9 | 5.2 | 10.5 | 15.7 | 20.0 |
| 32 | 1.4 | 4.6 | 9.4 | 14.9 | 18.9 |
| 33 | 1.1 | 3.7 | 8.2 | 13.8 | 17.7 |
| 34 | 1.0 | 5.2 | 10.8 | 17.3 | 21.7 |
| 35 | 1.2 | 2.8 | 5.8 | 9.8 | 12.6 |
| 36 | 0.9 | 2.7 | 5.5 | 9.3 | 11.7 |
| 37 | 0.5 | 1.4 | 2.4 | 4.0 | 5.6 |
| 38 | 1.1 | 3.2 | 6.0 | 9.0 | 11.2 |
| 39 | 1.4 | 4.2 | 8.1 | 13.0 | 16.0 |
| Comparative Example 1 | 0.3 | 0.8 | 1.2 | 1.7 | 2.2 |

### Example 5. TDD Systems

Two different transdermal drug delivery (TDD) systems (TDD Systems 40-41) were prepared each containing estradiol hemihydrate (CAS Number 35380-71-3, molecular formula = C₁₈H₂₄O₂ .0.5 H₂O) in a different drug in adhesive (DIA) formulation. For each TDD system the individual formulation components were combined with a blended adhesive consisting of 26 wt.% (dry basis) isooctyl acrylate/acrylamide/vinyl acetate (75/5/20) adhesive copolymer (35% solids in a solvent system of about 70/30 ethyl acetate/ methanol) and 74 wt.% (dry basis) AC7-4302 silicone adhesive (60% solids in ethyl acetate). The formulation mixture was gently agitated with a laboratory roller mixer to achieve a uniform distribution of the formulation components. The formulation was then knife coated onto a ScotchPak™ 9744 polyester film release liner (available from 3M Company, Maplewood, MN). The coated liner was oven dried (10 minutes at 60 °C) and then laminated onto a CoTran™ 9722 polyolefin monolayer film backing (available from 3M Company). The dry coating weight for each of the TDD Systems 40-41 was about 10 mg/cm². The finished TDD systems were die cut as individual circles with adhesive containing surface areas of 1 cm². The nominal concentrations of the formulation components in each finished TDD system are listed in Table 5. For Comparative Example 2, a TDD system was prepared as described above in which no levulinic acid was incorporated in the DIA formulation.

**Table 5**

| TDD System Number | Concentration in the Adhesive Formulation of the Finished TDD System (Percent by Weight) | | | | |
|---|---|---|---|---|---|
| | Estradiol Hemihydrate | Levulinic Acid | Polyvinylpyrrolidone (PVP) K-30 | Oleyl Alcohol | Dipropylene Glycol |
| Comparative Example 2 | 1.6% | 0% | 7.5% | 6.0% | 8.0% |
| 40 | 1.6% | 2.0% | 7.5% | 6.0% | 8.0% |
| 41 | 1.6% | 4.0% | 7.5% | 6.0% | 8.0% |

### Example 6. Skin Permeation Study

*In vitro* skin permeation of estadiol hemihydrate from each TDD system of Example 5 was determined using the same procedure as described in Example 2. The only difference was the sampling times, which were changed to 8 hours, 24 hours, 48 hours and 72 hours. The cumulative amount of estradiol hemihydrate delivered from the TDD system to the receptor compartment (µg) was determined at each time point. For each system a total of 4 replicates were analyzed and the mean value is reported in Table 6.

**Table 6.**

| TDD System Number | Cumulative Amount of Estradiol Hemihydrate (µg) Delivered to the Receptor Compartment at Sampling Timepoints (mean value reported with n=4) | | | |
|---|---|---|---|---|
| | 8 hours | 24 hours | 48 hours | 72 hours |
| Comparative Example 2 | 1.8 | 8.0 | 15.8 | 22.2 |
| 40 | 3.3 | 14.4 | 26.2 | 34.7 |
| 41 | 3.5 | 15.5 | 28.6 | 38.5 |

## Claims

1. A transdermal drug delivery system comprising:
a composition comprising:
(a) an effective amount of estradiol or a salt or a solvate or hydrate thereof; and
(b) levulinic acid in an amount suitable to enhance the transdermal permeation of estradiol, and
(c) a pharmaceutically acceptable carrier.

2. A transdermal drug delivery system according to Claim 1 wherein the carrier is an adhesive matrix.

3. A transdermal drug delivery system according to Claim 2 wherein the adhesive matrix comprises an acrylate copolymer.

4. A transdermal drug delivery system according to Claim 2 or 3 and further comprising a backing film.

5. A transdermal drug delivery system according to Claim 4 having a surface area of about 2 cm² to about 1.0 cm².

6. A transdermal drug delivery system according to any one of Claims 2 to 5 wherein the adhesive matrix is a reservoir layer and further comprising a skin-contacting adhesive layer that is distinct from the reservoir layer.

7. A transdermal drug delivery system according to Claim 6 wherein the skin-contacting adhesive layer comprises a silicone adhesive.

8. A transdermal drug delivery system according to Claim 1, wherein the carrier is a semi-solid dosage form.

9. A transdermal drug delivery system according to Claim 8 wherein the carrier is selected from the group consisting of gel, cream, ointment, solution, suspension, and paste.

10. A transdermal drug delivery system according to any of the preceding Claims and further comprising a solubilizing agent.

11. A transdermal drug delivery system according to Claim 10 wherein the solubilizing agent is selected from the group consisting of butylene glycol, diethylene glycol methyl ether, dipropylene glycol, propylene glycol, and mixtures thereof.

12. A transdermal drug delivery system according to Claim 10 wherein the solubilizing agent is dipropylene glycol.

13. A transdermal drug delivery system according to any one of Claims 10 to 12 wherein the amount of solubilizing agent is between about 2 and about 6 percent by weight of the composition.

14. A transdermal drug delivery system according to any of the preceding Claims wherein the estradiol is homogeneously dispersed within the composition.

15. A transdermal drug delivery system according to any of the preceding Claims and further comprising one or more additional permeation enhancers selected from the group consisting of lauryl lactate, oleic acid, and mixtures thereof.

16. A transdermal drug delivery system according to Claim 15 wherein the amount of additional permeation enhancer is between about 4 and about 8 percent by weight of the composition.

17. A transdermal drug delivery system according to any of the preceding Claims wherein the amount of estradiol is between about 2 and about 5 percent by weight of the composition.

18. A transdermal drug delivery system according to any of the preceding Claims wherein the estradiol or a salt or a solvate or hydrate thereof is estradiol hemihydrate.

19. A transdermal drug delivery system according to any of the preceding Claims wherein the amount of levulinic acid is between 5 and 25 percent by weight of the composition.

## Patentansprüche

1. System zur transdermalen Arzneimittelabgabe, umfassend:
eine Zusammensetzung, die umfasst:
(a) eine wirksame Menge von Estradiol oder einem Salz oder einem Solvat oder Hydrat davon; und
(b) Lävulinsäure in einer Menge, die geeignet ist, um die transdermale Permeation von Estradiol zu verstärken, und
(c) einen pharmazeutisch verträglichen Träger.

2. System zur transdermalen Arzneimittelabgabe nach Anspruch 1, wobei der Träger eine Klebstoffmatrix ist.

3. System zur transdermalen Arzneimittelabgabe nach Anspruch 2, wobei die Klebstoffmatrix ein Acrylatcopolymer umfasst.

4. System zur transdermalen Arzneimittelabgabe nach Anspruch 2 oder 3, ferner eine Trägerfolie umfassend.

5. System zur transdermalen Arzneimittelabgabe nach Anspruch 4 mit einer Oberfläche von etwa 2 cm² bis etwa 10 cm².

6. System zur transdermalen Arzneimittelabgabe nach einem der Ansprüche 2 bis 5, wobei die Klebstoffmatrix eine Reservoirschicht ist und ferner eine hautkontaktierende Klebstoffschicht umfasst, die sich von der Reservoirschicht unterscheidet.

7. System zur transdermalen Arzneimittelabgabe nach Anspruch 6, wobei die hautkontaktierende Klebstoffschicht einen Silikonklebstoff umfasst.

8. System zur transdermalen Arzneimittelabgabe nach Anspruch 1, wobei der Träger eine halbfeste Dosierungsform darstellt.

9. System zur transdermalen Arzneimittelabgabe nach Anspruch 8, wobei der Träger aus der Gruppe bestehend aus Gel, Creme, Salbe, Lösung, Suspension und Paste ausgewählt wird.

10. System zur transdermalen Arzneimittelabgabe nach einem der vorangehenden Ansprüche, ferner ein Lösungshilfsmittel umfassend.

11. System zur transdermalen Arzneimittelabgabe nach Anspruch 10, wobei das Lösungshilfsmittel aus der Gruppe, bestehend aus Butylenglycol, Diethylenglycolmethylether, Dipropylenglycol, Propylenglycol und Mischungen davon, ausgewählt ist.

12. System zur transdermalen Arzneimittelabgabe nach Anspruch 10, wobei das Lösungshilfsmittel Dipropylenglycol ist.

13. System zur transdermalen Arzneimittelabgabe nach einem der Ansprüche 10 bis 12, wobei die Menge an Lösungshilfsmittel zwischen etwa 2 und etwa 6 Gewichtsprozent der Zusammensetzung liegt.

14. System zur transdermalen Arzneimittelabgabe nach einem der vorangehenden Ansprüche, wobei das Estradiol innerhalb der Zusammensetzung homogen verteilt ist.

15. System zur transdermalen Arzneimittelabgabe nach einem der vorangehenden Ansprüche, ferner einen oder mehrere zusätzliche Permeationsverstärker umfassend, die aus der Gruppe, bestehend aus Lauryllactat, Oleinsäure und Mischungen davon, ausgewählt sind.

16. System zur transdermalen Arzneimittelabgabe nach Anspruch 15, wobei die Menge an zusätzlichem Permeationsverstärker zwischen etwa 4 und etwa 8 Gewichtsprozent der Zusammensetzung liegt.

17. System zur transdermalen Arzneimittelabgabe nach einem der vorangehenden Ansprüche, wobei die Menge an Estradiol zwischen etwa 2 und etwa 5 Gewichtsprozent der Zusammensetzung liegt.

18. System zur transdermalen Arzneimittelabgabe nach einem der vorangehenden Ansprüche, wobei das Estradiol oder ein Salz oder ein Solvat oder Hydrat davon Estradiolhemihydrat ist.

19. System zur transdermalen Arzneimittelabgabe nach einem der vorangehenden Ansprüche, wobei die Menge an Lävulinsäure zwischen 5 und 25 Gewichtsprozent der Zusammensetzung liegt.

## Revendications

1. Système d'administration transdermique de médicament, comprenant:
une composition comprenant:
(a) une quantité efficace d'oestradiol ou d'un sel ou d'un solvate ou hydrate de celui-ci; et
(b) de l'acide lévulinique en une quantité appropriée pour améliorer la perméation transdermique de l'oestradiol, et
(c) un véhicule acceptable sur le plan pharmaceutique.

2. Système d'administration transdermique de médicament selon la revendication 1, dans lequel le véhicule est une matrice adhésive.

3. Système d'administration transdermique de médicament selon la revendication 2, dans lequel la matrice adhésive comprend un copolymère acrylate.

4. Système d'administration transdermique de médicament selon la revendication 2 ou 3, et comprenant en outre un film de support.

5. Système d'administration transdermique de médicament selon la revendication 4, possédant une superficie d'environ 2 cm² à environ 10 cm².

6. Système d'administration transdermique de médicament selon l'une quelconque des revendications 2 à 5, dans lequel la matrice adhésive est une couche réservoir et comprenant en outre une couche adhésive de contact avec la peau qui est distincte de la couche réservoir.

7. Système d'administration transdermique de médicament selon la revendication 6, dans lequel la couche adhésive de contact avec la peau comprend un adhésif de silicone.

8. Système d'administration transdermique de médicament selon la revendication 1, dans lequel le véhicule est une forme pharmaceutique semi-solide.

9. Système d'administration transdermique de médicament selon la revendication 8, dans lequel le véhicule est choisi dans le groupe constitué de gel, crème, pommade, solution, suspension et pâte.

10. Système d'administration transdermique de médicament selon l'une quelconque des revendications précédentes, comprenant en outre un agent de solubilisation.

11. Système d'administration transdermique de médicament selon la revendication 10, dans lequel l'agent de solubilisation est choisi dans le groupe constitué de butylène glycol, éther méthylique de diéthylène glycol, dipropylène glycol, propylène glycol, et des mélanges de ceux-ci.

12. Système d'administration transdermique de médicament selon la revendication 10, dans lequel l'agent de solubilisation est le dipropylène glycol.

13. Système d'administration transdermique de médicament selon l'une quelconque des revendications 10 à 12, dans lequel la quantité d'agent de solubilisation est comprise entre environ 2 et environ 6 pour cent en poids de la composition.

14. Système d'administration transdermique de médicament selon l'une quelconque des revendications précédentes, dans lequel l'oestradiol est homogènement dispersé au sein de la composition.

15. Système d'administration transdermique de médicament selon l'une quelconque des revendications précédentes, comprenant en outre un ou plusieurs agents améliorant la perméation supplémentaires choisis dans le groupe constitué de lactate de lauryle, acide oléique, et des mélanges de ceux-ci.

16. Système d'administration transdermique de médicament selon la revendication 15, dans lequel la quantité d'agent améliorant la perméation supplémentaire est comprise entre environ 4 et environ 8 pour cent en poids de la composition.

17. Système d'administration transdermique de médicament selon l'une quelconque des revendications précédentes, dans lequel la quantité d'oestradiol est comprise entre environ 2 et environ 5 pour cent en poids de la composition.

18. Système d'administration transdermique de médicament selon l'une quelconque des revendications précédentes, dans lequel l'oestradiol ou un sel ou un solvate ou hydrate de celui-ci est l'hémihydrate d'oestradiol.

19. Système d'administration transdermique de médicament selon l'une quelconque des revendications précédentes, dans lequel la quantité d'acide lévulinique est comprise entre 5 et 25 pour cent en poids de la composition.
